# EUROPEAN PATENT APPLICATION

(11) **EP 4 154 837 A1**
(43) Date of publication of application: **29.03.2023**
(21) Application number: 22196844.9
(22) Date of filing: 21.09.2022
(51) Int. Cl.: A61B 34/30, A61B 34/00

(54) **SURGICAL ROBOTIC SYSTEM SETUP**

(30) Priority: 22.09.2021 US 202163246805 P; 17.08.2022 US 202217889912
(71) Applicant: Covidien LP, Mansfield, MA 02048 (US)
(72) Inventor: ROCKROHR, Brian A., Guilford, 06473 (US); LOSCHAK, Paul M., Cambridge, 02210 (US); HAGN, Ulrich, 82234 Wessling (DE); KAPADIA, Jaimeen V., Boston, 02210 (US); KONIETSCHKE, Rainer C., 82234 Wessling (DE)
(74) Representative: Maschio, Antonio

(57) **Abstract**

A surgical robotic system having multiple robotic arm carts and a surgeon console including a processor and a memory. The memory including instructions stored thereon, which when executed by the processor cause the surgical robotic system to receive data indicating a first pose of the first movable cart and a second pose of the second movable cart and determine a relative location of the first movable cart and the second movable cart based on the first pose and the second pose.

## Description

### CROSS-REFERENCE TO RELATED APPLICATIONS

The present application claims the benefit of and priority to U.S. Provisional Patent Application Serial No. 63/246,805, filed on September 22, 2021, the entire content of which being hereby incorporated by reference.

### BACKGROUND

Surgical robotic systems are currently being used in minimally invasive medical procedures. Some surgical robotic systems include a surgeon console controlling a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body.

While performing surgical procedures with multiple robotic arms that are supported on untethered movable surgical carts the relative positioning and operational status of each robotic arm can be difficult for users to associate with the actual movable cart.

### SUMMARY

According to one embodiment of the present disclosure, a surgical robotic system is disclosed. The surgical robotic system includes a first movable cart, a second movable cart, and a surgical console. The surgeon console includes a processor and a memory. Each of the movable carts include a robotic arm. The memory includes instructions stored thereon, which, when executed by the processor, cause the surgical robotic system to receive data indicating a first pose of the first movable cart and a second pose of the second movable cart and determine a relative location between the first movable cart and the second movable cart based on the first pose and the second pose.

In an aspect of the present disclosure, the surgical console may further include a display configured to display a graphical user interface having a plurality of graphical representations each of which corresponds to the first movable cart and the second movable cart. The instructions, when executed by the processor, may further cause the surgical robotic system to determine a position registration of the first cart based on the relative location between the first movable cart and the second movable cart and display the graphical representation of the first movable cart based on the determined position registration.

In another aspect of the present disclosure, the system may further include a plurality of directional buttons configured to indicate, as the data, a direction from which the first movable cart approaches the second movable cart.

In yet another aspect of the present disclosure, data indicating a first pose of the first movable cart and a second pose of the second movable cart may include user entered pose information.

In a further aspect of the present disclosure, the instructions, when executed by the processor, may further cause the surgical robotic system to automatically command each of the first movable cart and the second movable cart to move through a plurality of translation trajectories in an orthogonal direction and receive user input, as the data, correlating with tracking movement of each of the first movable cart and the second movable cart through the plurality of translation trajectories.

In yet a further aspect of the present disclosure, the first movable cart and the second movable cart each further include a sensor configured to measure travel and direction of the respective movable cart. The instructions, when executed by the processor, may further cause the surgical robotic system to generate a movement path, as the data, of each of the first movable cart and the second movable cart based on the sensed travel and direction of each of the first movable cart and the second movable cart.

In another aspect of the present disclosure, the first movable cart may further include a telescoping arm. The telescoping arm may include a plurality of joints including a respective plurality of encoders configured to provide signal indicating a distance and a sensor configured to sense contact of a predefined spatial location of the second movable cart by the telescoping arm. The instructions, when executed by the processor, may further cause the surgical robotic system to determine, as the data, the first pose of the first movable cart and the second pose of the second movable cart based on the sensed contact of the predefined spatial location of the second movable cart by the telescoping arm, the indicated distance, and the predefined spatial location.

In yet another aspect of the present disclosure, the first movable cart may include a first end effector. The second movable cart may include a second end effector. The instructions, when executed by the processor, may further cause the surgical robotic system to display, on the display, a predefined trajectory on the display for each of the first end effector and the second end effector; and receive input, as the data, correlating with tracking the predefined trajectory for each of the first end effector and the second end effector.

In a further aspect of the present disclosure, the system may further include an imaging device configured to capture images of a surgical site, including a first end effector of the first movable cart and a second end effector of the second movable cart. The data includes the captured image. The instructions, when executed by the processor, may further cause the surgical robotic system to identify the first end effector and the second end effector based on a computer vision algorithm; determine a relative pose between the first end effector and the second end effector based on the computer vision algorithm; determine the pose of the first movable cart and the second movable cart based on inverse kinematic mapping of the determined relative pose between the first end effector and the second end effector; and determine a position registration of the first cart based on the determined pose of the first movable cart and the second movable cart.

In yet a further aspect of the present disclosure, the first movable cart may further include a distance sensor configured to sense a distance between each of the movable carts. The instructions, when executed by the processor, may further cause the surgical robotic system to determine, as the data, the first pose of the first movable cart and the second pose of the second movable cart based on the sensed distance.

In another aspect of the present disclosure, the system may further include an imaging device configured to capture an image including each of the first movable cart and the second movable cart. Each of the first movable cart and the second movable cart may include a marker. The instructions, when executed by the processor, may further cause the surgical robotic system to detect each of the markers in the image; generate a point cloud of the first movable cart and the second movable cart in an operating room; and identify each of the first movable cart and the second movable cart using image segmentation. The data may include the point cloud and the identified first movable cart and the second movable cart.

In yet another aspect of the present disclosure, the first movable cart and the second movable cart each may include a spatial position sensor configured to sense a spatial position of the respective of the movable cart. The instructions, when executed by the processor, may further cause the surgical robotic system to sense, by the spatial position sensor, the spatial position of the first movable cart and the second movable cart and determine, as the data, the first pose of the first movable cart and the second pose of the second movable cart based on the sensed spatial position of the first movable cart and the second movable cart.

According to one embodiment of the present disclosure, a computer-implemented method for controlling a surgical robotic system is presented. The computer-implemented method includes receiving data indicating a first pose of a first movable cart and a second pose of a second movable cart and determining a relative location between the first movable cart and the second movable cart based on the first pose and the second pose. Each of the movable carts include a robotic arm.

In yet a further aspect of the present disclosure, the method may further include determining a position registration of the first cart based on the relative location between the first movable cart and the second movable cart and displaying a graphical representation of the first movable cart based on the determined position registration on a display of a surgical console. The display may be configured to display a graphical user interface having a plurality of graphical representations each of which corresponds to the first movable cart and the second movable cart.

In another aspect of the present disclosure, the method may further include automatically commanding each of the first movable cart and the second movable cart to move through a plurality of translation trajectories in an orthogonal direction; and receiving user input, as the data, correlating with tracking the movement of each of the first movable cart and the second movable cart through the plurality of translation trajectories.

In yet another aspect of the present disclosure, the method may further include contacting a predefined spatial location of the second movable cart by a telescoping arm of the first movable cart. The telescoping arm includes a plurality of joints including a respective plurality of encoders configured to provide signal indicating a distance. The method may further include a sensor configured to sense contact of the telescoping arm with another movable cart; sensing, by the sensor, the contact of the telescoping arm with the second movable cart; sensing, by the plurality of encoders, the distance and relative orientation between the first movable cart and the second movable cart; and determining, as the data, the first pose of the first movable cart and the second pose of the second movable cart based on the sensed distance and the predefined spatial location.

In a further aspect of the present disclosure, the method may further include capturing an image of a surgical site by an imaging device as the data, the image including a first end effector of the first movable cart and a second end effector of the second movable cart; identifying the first end effector and the second end effector based on a computer vision algorithm; determining a relative pose between the first end effector and the second end effector based on the computer vision algorithm; determining the pose of the first movable cart and the second movable cart based on inverse kinematic mapping of the determined relative pose between the first end effector and the second end effector; and determining a position registration of the first cart based on the determined pose of the first movable cart and the second movable cart.

In yet a further aspect of the present disclosure, the method may further include sensing, by a spatial position sensor, the spatial position of each of the first movable cart and the second movable cart, wherein the spatial position sensor is and determine, as the data, the first pose of the first movable cart and the second pose of the second movable cart based on the sensed spatial position of the first movable cart and the second movable cart.

In another aspect of the present disclosure, the method may further include sensing by first sensor a first travel and first direction of the first movable cart, sensing by second sensor a second travel and second direction of the second movable cart, and generating a movement path, as the data, of each of the first movable cart and the second movable cart based on the sensed travel and direction of each of the first movable cart and the second movable cart.

According to one embodiment of the present disclosure, a non-transitory computer-readable medium storing instructions which, when executed by a processor, cause the processor to perform a method for controlling a surgical robotic system is presented. The method includes receiving data indicating a first pose of a first movable cart and a second pose of a second movable cart, and determining a relative location between the first movable cart and the second movable cart based on the first pose and the second pose. Each of the movable carts includes a robotic arm.

### BRIEF DESCRIPTION OF THE DRAWINGS

Various aspects of the present disclosure are described herein with reference to the drawings wherein:
FIG. 1 is a schematic illustration of a surgical robotic system including a control tower, a console, and one or more surgical robotic arms each disposed on a movable cart according to an aspect of the present disclosure;
FIG. 2 is a perspective view of a surgical robotic arm of the surgical robotic system of FIG. 1 according to an aspect of the present disclosure;
FIG. 3 is a perspective view of a setup arm with the surgical robotic arm of the surgical robotic system of FIG. 1 according to an aspect of the present disclosure;
FIG. 4 is a schematic diagram of a computer architecture of the surgical robotic system of FIG. 1 according to an aspect of the present disclosure;
FIG. 5 is a plan schematic view of movable carts of FIG. 1 positioned about a surgical table according to an aspect of the present disclosure;
FIG. 6 is a graphical user interface displayed on a display of the surgeon console according to an embodiment of the present disclosure;
FIG. 7 is a perspective view of robotic arms of the movable carts of FIG. 1 according to an embodiment of the present disclosure; and
FIG. 8 is a flow chart of a method for controlling the surgical robotic system of FIG. 1 according to an embodiment of the present disclosure.

### DETAILED DESCRIPTION

The term "application" may include a computer program designed to perform functions, tasks, or activities for the benefit of a user (e.g., a clinician). Application may refer to, for example, software running locally or remotely, as a standalone program or in a web browser, or other software which would be understood by one skilled in the art to be an application. An application may run on a controller, or on a user device, including, for example, a mobile device, a personal computer, and/or a server system.

As will be described in detail below, the present disclosure is directed to a surgical robotic system, which includes a surgeon console, a control tower, and one or more movable carts having a surgical robotic arm coupled to a setup arm. The surgeon console receives user input through one or more interface devices, which are interpreted by the control tower as movement commands for moving the surgical robotic arm. The surgical robotic arm includes a controller, which is configured to process the movement command and to generate a torque command for activating one or more actuators of the robotic arm, which would, in turn, move the robotic arm in response to the movement command.

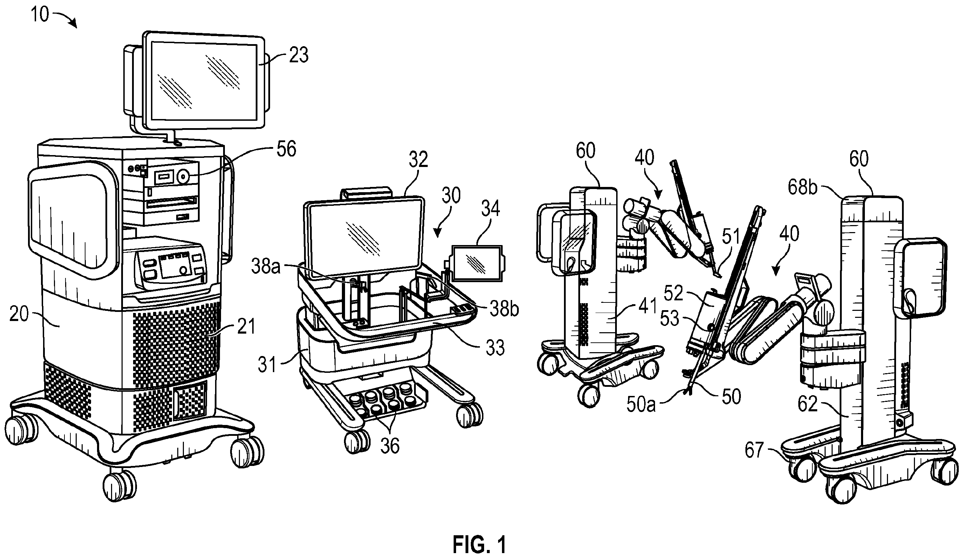

With reference to FIG. 1, a surgical robotic system 10 includes a control tower 20, which is connected to all of the components of the surgical robotic system 10 including a surgeon console 30 and one or more robotic arms 40. Each of the robotic arms 40 includes a surgical instrument 50 removably coupled thereto. Each of the robotic arms 40 is also coupled to a movable cart 60.

The surgical instrument 50 is configured for use during minimally invasive surgical procedures. In aspects, the surgical instrument 50 may be configured for open surgical procedures. In aspects, the surgical instrument 50 may be an endoscope, such as an endoscopic camera 51, configured to provide a video feed for the user. In further aspects, the surgical instrument 50 may be an electrosurgical forceps configured to seal tissue by compressing tissue between jaw members and applying electrosurgical current thereto. In yet further aspects, the surgical instrument 50 may be a surgical stapler including a pair of jaws configured to grasp and clamp tissue while deploying a plurality of tissue fasteners, e.g., staples, and cutting stapled tissue.

One of the robotic arms 40 may include the endoscopic camera 51 configured to capture video of the surgical site. The endoscopic camera 51 may be a stereoscopic endoscope configured to capture two side-by-side (i.e., left and right) images of the surgical site to produce a video stream of the surgical scene. The endoscopic camera 51 is coupled to a video processing device 56, which may be disposed within the control tower 20. The video processing device 56 may be any computing device as described below configured to receive the video feed from the endoscopic camera 51 perform the image processing based on the depth estimating algorithms of the present disclosure and output the processed video stream.

The surgeon console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs. The video processing device 56 is configured to process the video feed from the endoscopic camera 51 and to output a processed video stream on the first displays 32 of the surgeon console 30 and/or the display 23 of the control tower 20.

The surgeon console 30 also includes a plurality of user interface devices, such as foot pedals 36 and a pair of handle controllers 38a and 38b, which are used by a user to remotely control robotic arms 40. The surgeon console further includes an armrest 33 used to support the clinician's arms while operating the handle controllers 38a and 38b.

The control tower 20 includes a display 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the handle controllers 38a and 38b.

Each of the control tower 20, the surgeon console 30, and the robotic arm 40 includes a respective computer 21, 31, 41. The computers 21,31,41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

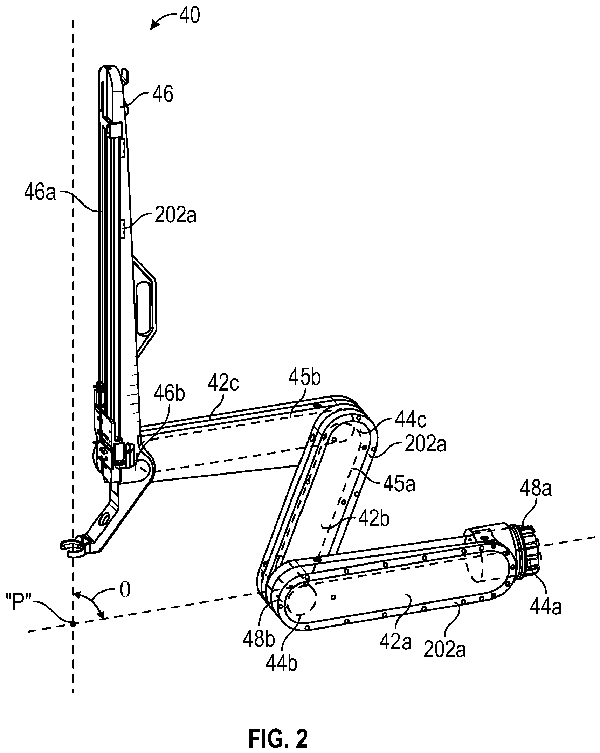

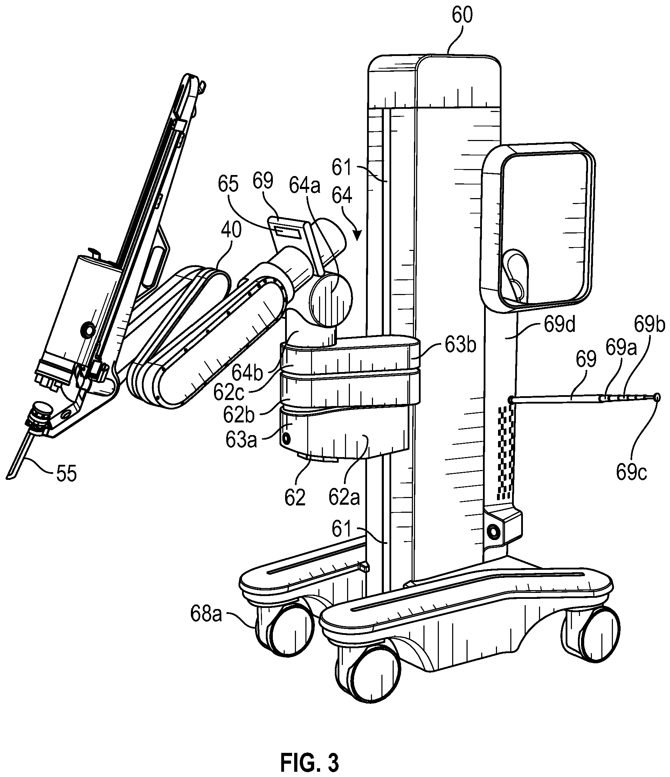

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display (not shown) for displaying information pertaining to the robotic arm 40.

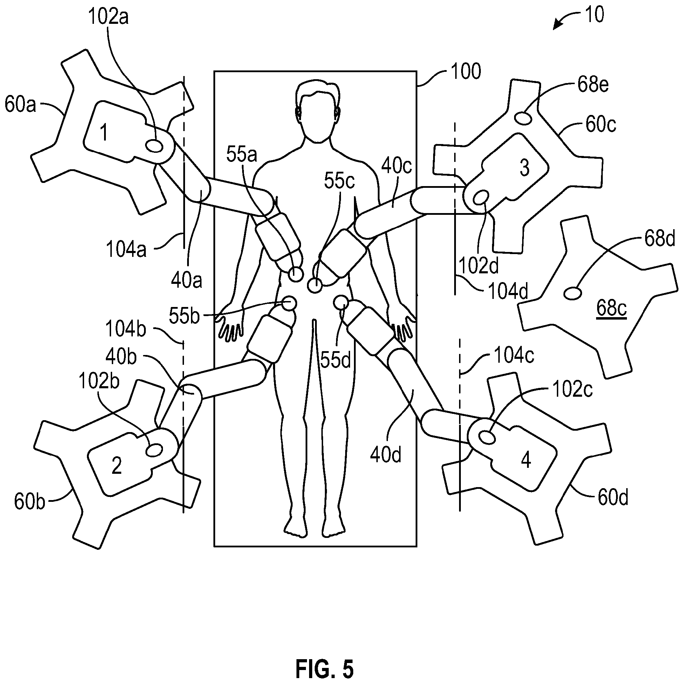

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In aspects, the robotic arm 40 may be coupled to a surgical table 100 (FIG. 5). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In aspects, some, or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effector) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic port 55 (FIG. 3) held by the holder 46.

The robotic arm 40 also includes a plurality of manual override buttons 53 (FIGS. 1 and 5) disposed on the IDU 52 and the setup arm 62, which may be used in a manual mode. The user may press one or more of the buttons 53 to move the component associated with the button 53.

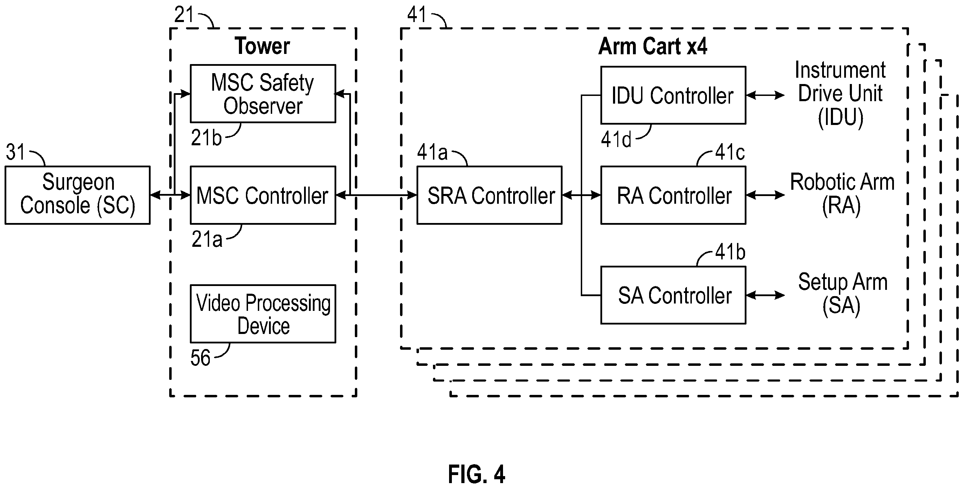

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled in response to a pose of the handle controller controlling the robotic arm 40, e.g., the handle controller 38a, which is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgeon console 30. The desired pose of the instrument 50 is relative to a fixed frame on the robotic arm 40. The pose of the handle controller 38a is then scaled by a scaling function executed by the controller 21a. In aspects, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

With reference to FIG. 5, the surgical robotic system 10 is setup around the surgical table 100. The surgical robotic system 10 includes movable carts 60a-d, which may be numbered "1" through "4."

During setup, each of the carts 60a-d are positioned around the surgical table 100. Position and orientation of the carts 60a-d depends on a plurality of factors, such as placement of a plurality of ports 55a-d, which in turn, depends on the surgery being performed. Once the port placement is determined, the ports 55a-d are inserted into the patient, and carts 60a-d are positioned to insert instruments 50 and the endoscopic camera 51 into corresponding ports 55a-d. Orientation of the carts 60a-d and their corresponding robotic arms 40 may be based on individual laser alignment patterns 104a-d. For a more detailed description of using alignment patterns to orient a plurality of movable carts 60 see International Application No. PCT/US2021/034125, titled "SURGICAL ROBOTIC SYSTEM USER INTERFACES", (published as WO 2021/247294), filed on May 26, 2021, the entire disclosure of which is incorporated by reference herein.

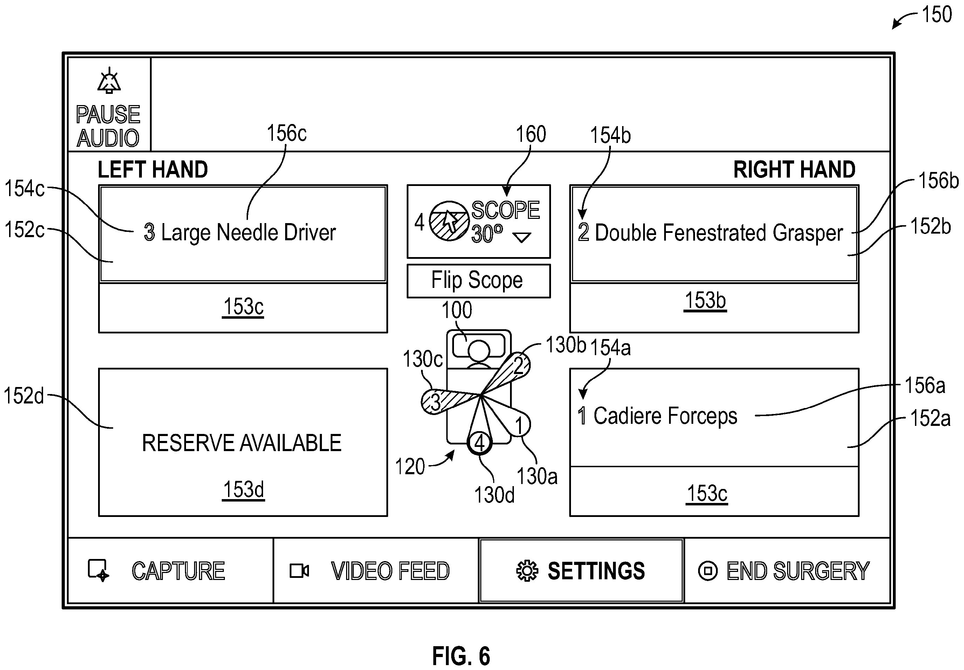

Once the movable carts 60a-d are placed at their desired positions, the surgeon or any other personnel may register each of the robotic arms 40 on a graphical user interface (GUI) 150, which may be displayed on the second display 34 or any other display of the surgical robotic system 10, e.g., displays 23 or 32. With reference to FIG. 6, the GUI 150 includes a plurality of regions 153a-d which include graphical representations 152a-c for each of the three robotic arms 40a-c, which are numbered "1"-"3" and a reserve graphical representation 152d. Each of the graphical representations 152a-c includes an identification number 154a-c and an instrument type 156a-c. The GUI 150 also includes an orientation indicator 160. The orientation indicator 160 shows rotation and pitch indication of the camera 51, which shows rotation and pitch indication of the camera 51 that is coupled to the robotic arm 40d numbered "4." The arms 60a-d may be automatically assigned to each of the graphical representations 152a-c, with the graphical representations 152a and 152b being controlled by the right-hand controller 38b and the graphical representations 152c and 152d being controlled by the left-hand controller 38a. However, the surgeon may move the instruments 50, i.e., robotic arms 60a-c between any of the four graphical representations 152a-d.

The GUI 150 also shows a bed map 120 showing a surgical table 100 and each of the robotic arms 40 represented as arrows 130a-d. Similar to the graphical representations 152a-c.

As noted above, the second display 34 is a touchscreen, which allows for moving the graphical representations 152a-d between the regions 153a-d by pressing, holding, and moving or using any other suitable touch gesture, e.g., moving the graphical representation 152a from the region 153a to any of the other regions 153b-d. This assigns the instrument to a desired one of the hand controllers 38a and 38b, designated as "LEFT HAND" and "RIGHT HAND," respectively. As the icons are moved between any of the graphical representations 152a-c, the user can confirm the actual physical location of the instruments 50 and their corresponding robotic arms 40.

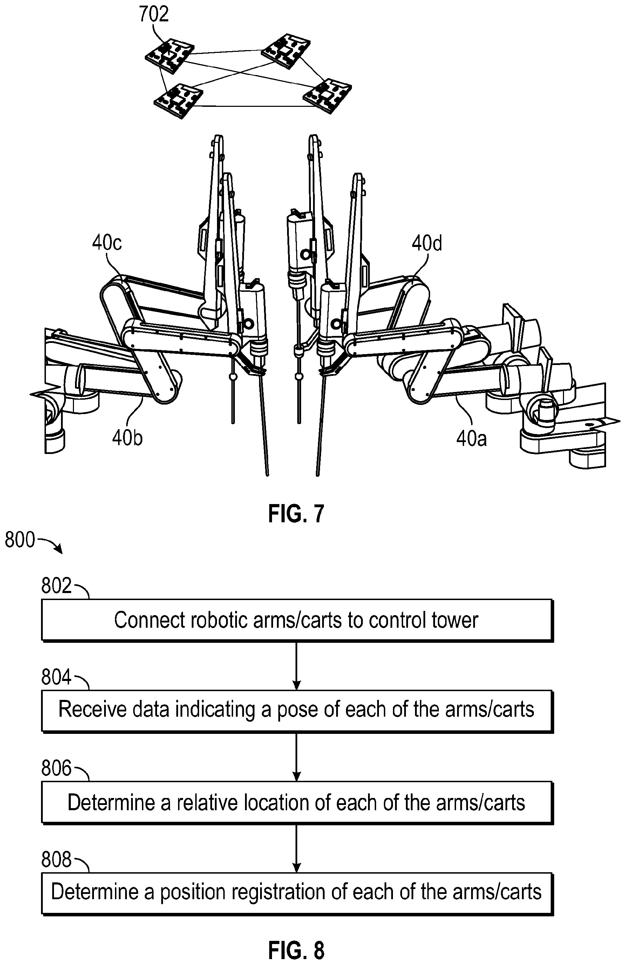

With reference to FIG. 8, a method 800 of registering a plurality of movable carts 60a-d is shown. Generally, surgical robotic systems 10 can feature robotic arms 40 mounted to separate movable carts 60a-d where each movable cart can be manipulated independently of the others. The surgical robotic system 10 then may use positioning and/or orientation information between the carts to understand the relative locations of each of the robotic arms 40. The surgical robotic system 10 acquires this information by "registration."

Surgeons require some form of registration between robotic arms 40 to operate the surgical robotic system 10. Understanding orientation differences between robotic arms 40 provides a foundation for algorithms such as hand-eye coordination, which enables the clinician's hand motions to drive instruments in the correct directions from the clinician's perspective. Understanding position differences between robotic arms 40 enables other capabilities such as collision avoidance. Performing automated tasks involving multiple robotic arms 40 also requires position registration.

The method 800 includes connecting each of the movable carts 60a-d to the control tower 20 at step 802 and receiving data indicating a pose of each of the movable carts 60a-d at step 804. The pose of each of the movable carts 60a-d includes the position and/or orientation of the movable carts 60a-d and/or the position and/or orientation of the robotic arms 40 of each of the movable carts.

In an aspect, the movable carts 60a-d may include a user interface for the user to input directional information. For example, a set of directional buttons could denote North/South/East/West, which represent the location of each arm with respect to its neighbors. This approach provides a general direction from which a movable cart 60a approaches the other movable carts 60b-d and a patient.

In another aspect, the user may triangulate movable carts 60a-d orientations and/or positions using an interface such as the GUI 150 (FIG. 6). Using the interface, the user would direct all movable carts 60a-d towards a single target for orientation info. If position info is desired the user could then direct all movable carts 60a-d to a second target, and then to a third target, and so on. The target object could be other carts or a fiducial marker on a patient, a Surgical table 100 or other component of the surgical robotic system 10. In aspects, the interface may include a rotational module (such as a laser module), a joystick, a lever, a label and/or specific feature that is temporarily and/or permanently attached to a rotational part of the robotic arm 40 (e.g., a label on a sterile interface module), a rotational knob on a pictogram of the patient, and/or other means of indicating direction.

In aspects, the user may directly enter pose information into the interface (e.g., the graphical user interface of FIG. 6). The user may directly indicate for each movable cart 60a-d, the movable cart's location with respect to neighboring movable carts 60a-d and/or robotic arms 40.

In a further aspect, data indicating the pose of each of the movable carts 60a-d may be determined by commanding each movable carts 60a-d to move through several small translation trajectories in orthogonal directions, which the user mimics at the user interface. Similarly, the clinician at the console 30 may control an end effector 50a of a surgical instrument 50 to navigate along a trajectory that is pre-defined on the first displays 32 of the surgeon console 30 and/or the display 23 of the control tower 20.

Alternatively, integrated optical sensors in the moveable cart 60a-d may be directed towards the floor and apply image flow. Optical flow is the pattern of apparent motion of objects between consecutive frames of sequence, caused by the relative movement between the object and imaging device. This can also be implemented using optical tracking devices that rely on optical flow and IMU to provide the translation and orientation information of the movable carts 60a-d.

With reference to FIG. 3, in another aspect, the pose of each of the movable carts 60a-d may be determined based on mechanical distance sensors disposed on each of the movable carts 60a-d. The movable carts 60a-d may include a telescoping arm with multiple joints 69b (e.g., spherical) with measured positions (e.g., by using encoders 69a and/or distance sensors). The user then moves a distal tip of the telescoping arm 69 and contact a predefined spatial location 69d of the neighboring movable cart 60a-d. The tip may include a contact sensor 69c configured to sense contact with an object, such as the predefined spatial location 69d of the neighboring movable cart 60a-d. By confirming contact through user input, the encoder values can be used to determine relative positions of neighboring movable carts 60a-d.

In another aspect, the pose of each of the movable carts 60a-d may be determined based on a measuring system integrated into the floor of the operating room (OR) and/or on a surface disposed on the floor of the OR. For example, the area around the surgical table 100 may be covered with a thin cover that integrates a measuring system to detect cart locations (e.g., a system that includes targets that are sensed by mechanical or optical means as they are passed). Examples include applying magnetic measurement principles, RFID, and ultra-wideband (UWB). UWB is a technology that enables peer-to-peer fine ranging using UWB transceivers 702 (FIG. 7) by performing time of flight measurements between the UWB transceivers 702 (e.g., based on the IEEE 802.15.4a standard) by sending a polling signal and receiving a response signal that includes a reply time. One of skill in the art would be familiar with how to implement UWB to implement a measuring system. For example, the robotic arms 40a-d may include UWB transceivers 702, enabling the fine ranging between each of the robotic arms 40a-d. Alternatively, the movable cart 60a-d may include a sensor configured to determine a distance based on the measuring system integrated into the floor and the passive measuring target is integrated into the thin cover (e.g., an optical sensor disposed on the movable cart 60a-d and optical grating on the floor). The targets may include barcodes, QR codes, and/or other graphical indicia, placed on the floor that are read by the sensor. Each barcode and/or QR can be unique so that the movable cart 60a-d can localize itself to the OR.

In another aspect, the pose of each of the movable carts 60a-d may be determined based on a computer vision algorithm. An imaging device, such as endoscopic camera 51, or other suitable camera, may be used to capture images (2D and/or 3D) of the surgical site, and/or the OR. The captured image would include the end effector 50a of the surgical instrument 50 of each of the movable carts 60a-d. The processor may then use a computer vision algorithm (such as image classification, object detection, object tracking, semantic segmentation, and/or instance segmentation, etc.) to identify the end effector 50a of the surgical instrument 50 (e.g., the end effector) of each of the movable carts 60a-d. The processor may then determine a relative pose between the end effector 50a of each of the surgical instruments 50 of each of the movable carts 60a-d based on the computer vision algorithm. The processor then determines the pose of the movable carts 60a-d based on inverse kinematic mapping of the determined relative pose between the end effectors 50a of the surgical instruments 50 of each of the movable carts 60a-d. The computer vision algorithm may include one or more convolutional neural networks configured to extract the features of the images based on training data.

In image classification, images relating to the various components of the surgical robotic system 10 may be labeled as different classes and used as training data. These classes may be utilized to classify and localize the various components of the surgical robotic system 10 and/or related OR objects for identification, including different robotic arms 40, movable carts 60a-d, and/or surgical instruments 50. This architecture may include various CNNs such as Alexnet, LeNet-5, VGG 16, DenseNet, and/or ResNet.

In object detection, input is scanned by the system for regions to classify and localize the various components of the surgical robotic system 10 and related OR objects, including different robotic arms 40, movable carts 60a-d, and surgical instruments 50. Object detection may be used for specialized tasks such as pose estimation to determine the orientation of the movable carts 60a-d. The architecture may include R-CNN (Region-based Convolutional Neural Network), Fast R-CNN, Faster R-CNN, and Region Proposal Network (RPN). This may further include Major Object Detection algorithms for shared computation of an input image, including Region-Based Fully Convolutional Networks (R-FCN), You Only Look Once (YOLO), and/or Single Shot MultiBox Detector (SSD).

In another aspect, the pose of each of the movable carts 60a-d may be determined based on a fiber optic shape sensor in the data/power cables of the movable cart 60a-d. A fiber optic shape sensor disposed in the data cables or power cables may be used to determine the position and orientation of each movable cart 60a-d relative to a control tower 20 (FIG. 1) and thus to each other. The fiber optic shape sensor enables a 3D reconstruction of the thin flexible optical fiber. The fiber optic shape sensor generally includes long thin flexible optical fiber has notches (gratings) along its length (not shown). A light box emits light down the flexible optical fiber and then receives the returned reflected light such that it is able to tell how far away each grating is. This information may be used to understand the curvature of the flexible optical fiber, which can then be used to calculate the location of any point along the fiber, such as the location of the moveable cart 60a-d attached to the other end. The fiber optic shape sensor can sense in a spatially continuous manner, providing information about the location and shape of the entire length of the instrument without the use of X-rays or ultrasound. The data generated by the fiber optic shape sensor may be plotted in real-time and displayed visually on a display to show the position and overall shape of the moveable cart 60a-d. This image can also be compared to known coordinates of locations within the OR, enabling clinicians to pair the visual reference from the tip of an instrument, such as an endoscope, with knowledge of how and where the rest of the instrument is positioned. An example sensor could be a fiber Bragg sensor.

In another aspect, the pose of each of the movable carts 60a-d may be determined based on a distance sensor 68b (FIG. 1) disposed on each of the movable carts 60a-d. The distance sensor 68b is configured to measure the distance between the movable cart 60a-d that it is disposed on and the other movable carts 60a-d. The distance sensor 68b may include LIDAR, ultrasound, RADAR, infrared, structured light, a depth camera, and/or electromagnetic sensors. For example, the distance between any two movable carts may be determined by an electromagnetic sensor configured to generate an electromagnetic signal and sense a distance based on a received return signal. Alternatively, a subset of movable carts 60a-d (not every cart) may include distance sensors 68b installed which are able to sense the distances of each movable cart 60a-d with respect to each other. It is contemplated that a distance sensors 68b may be used to register the location of the Surgical table 100. The pose of each of the movable carts 60a-d may be determined based on generating a planar field of view or a 3D depth map to register common features that are observed by each of the distance sensors 68b of the movable carts 60a-d. For example, the common feature could be an object or an existing fixture in the OR such as lights and/or Surgical table 100s. Multiple sensors may be placed at different configurable heights and/or angles to minimize interference.

In a further aspect, the pose of each of the movable carts 60a-d may be determined based on a distance measurement system 68c not mounted on the movable carts 60a-d (FIG. 5). The distance measurement system 68c may be disposed anywhere in the OR and provide measurements of movable cart 60a-d locations. The distance measurement system 68c may be mounted to a centralized control tower 20 (FIG. 1), or to a separate movable unit (e.g., a handheld device). The distance measurement system 68c may detect markers 68e which are attached to the movable carts 60a-d, such as reflective markers with time-of-flight sensors and/or optical tracking markers for recognizing visual feature patterns. The markers 68e may be attached to each movable cart 60a-d. The depth measurement system 68c may use an imaging device capable of depth sensing, such as an RGB-D camera, to generate a point cloud of the components of the surgical robotic system 10 (FIG. 1) in the OR and then use an image segmentation algorithm to identify each movable cart 60a-d.

For example, the distance sensor 68b may be mounted on a mechanical platform that may be rotated (between about 0 to about 360 degrees and/or multi-rotational) to change the field of view of the sensor so that it can cover the complete area of the OR. The mechanical platform may include an actuator, that would have a sensor, such as an encoder, to measure the angle of the distance sensor 68b and use that information to generate a map of the OR. It is contemplated that the mechanical platform could be also actuated in other degrees of freedom (such as XYZ) to expand its field of view.

In a further aspect, the pose of each of the movable carts 60a-d may be determined based on a networked distance measurement. The pose (i.e., the position and/or orientation) of each movable cart 60a-d in the OR may be determined based on a network broadcast in the OR. Each of the movable carts 60a-d may include a wireless card and communicate across a 5G network. Various wireless signal characteristics may be used to determine position info, for example, signal-to-noise ratio, and/or signal strength. In aspects, GPS, Bluetooth, and ultra-wideband (UWB) may be used to determine the pose of each movable cart 60a-d.

In yet a further aspect, sensors 202a (FIG. 2) may be integrated into moving portions of the robotic arms 40. This may allow increased registration accuracy because motorized robotic arm motion is known and could be used to refine the registration. When measuring over a longer period of time and while moving the sensors in a known way, static noise in the measurement can be reduced. The system can also have modules that can be attached to the patient, port, OR staff, and/surgical table 100.

With reference to FIG. 8, at step 806, the processor determines a relative location between each of the movable carts 60a-d (and/or robotic arms 40) based on the data which indicated the pose of each of the movable carts 60a-d.

At step 806, the processor determines a position registration for each of the movable carts 60a-d (and/or robotic arms 40) based on the relative location between the movable carts 60a-d (and/or robotic arms 40). In aspects, the graphical representation of the movable carts 60a-d may be displayed based on the determined position registration.

In an aspect, odometry may be used after initial position registration. For example, before setup, all of the movable carts 60a-d may be placed next to each other in such way that their relative position to each other is known (e.g., the movable carts 60a-d could be lined up next to each other with their sides touching each other or the carts are placed on markings on the floor). The movable carts 60a-d may include casters 67 (FIG. 1), which are equipped with sensors 68a (FIG. 3) suitable to measure travel and direction of each of the casters 67. By using the principle of odometry, this sensor data can be accumulated to generate the movement path of every movable cart 60a-d during setup. The actual positions and orientations of each movable cart 60a-d with respect to each other may be determined, which provides continuous registration.

Once the position registration of the movable carts 60a-d is determined, the position registration may be used to generate guidance for users during setup of the movable carts 60a-d by displaying advice recommending the best positioning of the movable carts 60a-d for access during the procedure. The patient habitus and the clinician preferences may be used as additional data by the processor when generating the guidance.

Further, the position registration of the movable carts 60a-d may be used for automated setup of the components of the surgical robotic system 10. For example, each movable cart 60a-d may include a self-driving capacity to automatically position and configure its setup arm for optimal access based on the position registration and at least one of surgeon preference, OR size, patient habitus, and/or type of surgery.

In aspects, the position registration of the movable carts 60a-d may be used for collision avoidance between components of the surgical robotic system 10. The distance measurement described above may also be used to sense if the two movable carts 60a-d are within a predefined distance (i.e., are getting too close to each other) or if a bedside assist is likely to get hit by a robotic arm 40. The surgical robotic system 10 can issue a notification (e.g., audio, visual, and/or haptic) and/or override the surgeon inputs to lower the speeds to minimize the effect of impact.

Whereas the movable carts have been used as an example component of the surgical robotic system 10, the methods described may be used for any component of the surgical robotic system 10. For example, the position registration of the movable carts 60a-d may be used for surgical table 100 tracking.

It will be understood that various modifications may be made to the aspects disclosed herein. In aspects, the relative position, angular orientation, and operational status of each robotic arm may also be simultaneously viewable on multiple displays. Therefore, the above description should not be construed as limiting, but merely as exemplifications of various aspects. Those skilled in the art will envision other modifications within the scope and spirit of the claims appended thereto.

The invention may be described by reference to the following numbered paragraphs:-1. A surgical robotic system comprising:
a first movable cart;
a second movable cart, wherein each of the movable carts includes a robotic arm; and
a surgeon console including:
   a processor; and
   a memory, including instructions stored thereon, which when executed by the processor cause the surgical robotic system to:
      receive data indicating a first pose of the first movable cart and a second pose of the second movable cart; and
      determine a location of the first movable cart relative to the second movable cart based on the first pose and the second pose.
2. The surgical robotic system according to paragraph 1, wherein the surgical console further includes a display configured to display a graphical user interface having a plurality of graphical representations each of which corresponds to the first movable cart and the second movable cart, and
   wherein the instructions, when executed by the processor, further cause the surgical robotic system to:
   determine a position registration of the first cart based on the location between the first movable cart and the second movable cart; and
   display the graphical representation of the first movable cart based on the determined position registration.
3. The surgical robotic system according to paragraph 1, wherein the system further comprises a plurality of directional buttons configured to indicate, as the data, a direction from which the first movable cart approaches the second movable cart.
4. The surgical robotic system according to paragraph 1, wherein the data indicating a first pose of the first movable cart and a second pose of the second movable cart includes user entered pose information.
5. The surgical robotic system according to paragraph 1, wherein the instructions, when executed by the processor, further cause the surgical robotic system to:
   automatically command each of the first movable cart and the second movable cart to move through a plurality of translation trajectories in an orthogonal direction; and
   receive user input, as the data, correlating with tracking movement of each of the first movable cart and the second movable cart through the plurality of translation trajectories.
6. The surgical robotic system according to paragraph 1, wherein the first movable cart and the second movable cart each further include a sensor configured to measure travel and direction of the respective movable cart, and
   wherein the instructions, when executed by the processor, further cause the surgical robotic system to:
   generate a movement path, as the data, of each of the first movable cart and the second movable cart based on the sensed travel and direction of each of the first movable cart and the second movable cart.
7. The surgical robotic system according to paragraph 1, wherein the first movable cart further includes a telescoping arm including:
   a plurality of joints including a respective plurality of encoders configured to provide signal indicating a distance; and
   a sensor configured to sense contact of a predefined spatial location of the second movable cart by the telescoping arm, and
      wherein the instructions, when executed by the processor, further cause the surgical robotic system to:
   determine, as the data, the first pose of the first movable cart and the second pose of the second movable cart based on the sensed contact of the predefined spatial location of the second movable cart by the telescoping arm, the indicated distance, and the predefined spatial location.
8. The surgical robotic system according to paragraph 2, wherein the first movable cart includes a first end effector, and
   wherein the second movable cart includes a second end effector, and
   wherein the instructions, when executed by the processor, further cause the surgical robotic system to:
      display, on the display, a predefined trajectory on the display for each of the first end effector and the second end effector; and
      receive input, as the data, correlating with tracking the predefined trajectory for each of the first end effector and the second end effector.
9. The surgical robotic system according to paragraph 1, further comprising an imaging device configured to capture images of a surgical site, including a first end effector of the first movable cart and a second end effector of the second movable cart,
   wherein the data includes the captured image; and
   wherein the instructions, when executed by the processor, further cause the surgical robotic system to:
      identify the first end effector and the second end effector based on a computer vision algorithm;
      determine a relative pose between the first end effector and the second end effector based on the computer vision algorithm;
      determine the pose of the first movable cart and the second movable cart based on inverse kinematic mapping of the determined relative pose between the first end effector and the second end effector; and
      determine a position registration of the first cart based on the determined pose of the first movable cart and the second movable cart.
10. The surgical robotic system according to paragraph 1, wherein the first movable cart further includes a distance sensor configured to sense a distance between each of the movable carts, and
   wherein the instructions, when executed by the processor, further cause the surgical robotic system to:
   determine, as the data, the first pose of the first movable cart and the second pose of the second movable cart based on the sensed distance.
11. The surgical robotic system according to paragraph 1, further comprising an imaging device configured to capture an image including each of the first movable cart and the second movable cart, wherein each of the first movable cart and the second movable cart includes a marker, and
   wherein the instructions, when executed by the processor, further cause the surgical robotic system to:
      detect each of the markers in the image;
      generate a point cloud of the first movable cart and the second movable cart in an operating room; and
      identify each of the first movable cart and the second movable cart using image segmentation,
   wherein the data includes the point cloud and the identified first movable cart and the second movable cart.
12. The surgical robotic system according to paragraph 1, wherein the first movable cart and the second movable cart each includes a spatial position sensor configured to sense a spatial position of the respective movable cart, and
   wherein the instructions, when executed by the processor, further cause the surgical robotic system to:
   sense, by the spatial position sensor, the spatial position of the first movable cart and the second movable cart; and
   determine, as the data, the first pose of the first movable cart and the second pose of the second movable cart based on the sensed spatial position of the first movable cart and the second movable cart.
13. A computer-implemented method for controlling a surgical robotic system, the method comprising:
   receiving data indicating a first pose of a first movable cart and a second pose of a second movable cart, wherein each of the movable carts includes a robotic arm; and
   determining a relative location between the first movable cart and the second movable cart based on the first pose and the second pose.
14. The method according to paragraph 13, further comprising:
   determining a position registration of the first cart based on the relative location between the first movable cart and the second movable cart; and
   displaying a graphical representation of the first movable cart based on the determined position registration on a display of a surgical console, wherein the display is configured to display a graphical user interface having a plurality of graphical representations, each of which corresponds to the first movable cart and the second movable cart.
15. The method according to paragraph 13, further comprising:
   automatically commanding each of the first movable cart and the second movable cart to move through a plurality of translation trajectories in an orthogonal direction; and
   receiving user input, as the data, correlating with tracking movement of each of the first movable cart and the second movable cart through the plurality of translation trajectories.
16. The method according to paragraph 13, further comprising:
   contacting a predefined spatial location of the second movable cart by a telescoping arm of the first movable cart, the telescoping arm including:
      a plurality of joints including a respective plurality of encoders configured to provide a signal indicating a distance; and
      a sensor configured to sense contact of the telescoping arm with another movable cart,
   sensing, by the sensor, the contact of the telescoping arm with the second movable cart;
   sensing, by the plurality of encoders, the distance between the first movable cart and the second movable cart; and
   determining, as the data, the first pose of the first movable cart and the second pose of the second movable cart based on the sensed distance and the predefined spatial location.
17. The method according to paragraph 13, further comprising:
   capturing an image of a surgical site by an imaging device as the data, the image including a first end effector of the first movable cart and a second end effector of the second movable cart,
   identifying the first end effector and the second end effector based on a computer vision algorithm;
   determining a relative pose between the first end effector and the second end effector based on the computer vision algorithm;
   determining the pose of the first movable cart and the second movable cart based on inverse kinematic mapping of the determined relative pose between the first end effector and the second end effector; and
   determining a position registration of the first cart based on the determined pose of the first movable cart and the second movable cart.
18. The method according to paragraph 13, further comprising:
   sensing, by a spatial position sensor, the spatial position of each of the first movable cart and the second movable cart, wherein the spatial position sensor is; and
   determine, as the data, the first pose of the first movable cart and the second pose of the second movable cart based on the sensed spatial position of the first movable cart and the second movable cart.
19. The method according to paragraph 13, further comprising:
   sensing by first sensor a first travel and first direction of the first movable cart;
   sensing by second sensor a second travel and second direction of the second movable cart; and
   generating a movement path, as the data, of each of the first movable cart and the second movable cart based on the sensed travel and direction of each of the first movable cart and the second movable cart.
20. A non-transitory computer-readable medium storing instructions which, when executed by a processor, cause the processor to perform a method for controlling a surgical robotic system, comprising:
   receiving data indicating a first pose of a first movable cart and a second pose of a second movable cart, wherein each of the movable carts includes a robotic arm; and
   determining a relative location between the first movable cart and the second movable cart based on the first pose and the second pose.

## Claims

1. A surgical robotic system comprising:
a first movable cart;
a second movable cart, wherein each of the movable carts includes a robotic arm; and
a surgeon console including:
a processor; and
a memory, including instructions stored thereon, which when executed by the processor cause the surgical robotic system to:
receive data indicating a first pose of the first movable cart and a second pose of the second movable cart; and
determine a location of the first movable cart relative to the second movable cart based on the first pose and the second pose.

2. The surgical robotic system according to claim 1, wherein the surgical console further includes a display configured to display a graphical user interface having a plurality of graphical representations each of which corresponds to the first movable cart and the second movable cart, and
wherein the instructions, when executed by the processor, further cause the surgical robotic system to:
determine a position registration of the first cart based on the location between the first movable cart and the second movable cart; and
display the graphical representation of the first movable cart based on the determined position registration.

3. The surgical robotic system according to claim 1 or claim 2, wherein the system further comprises a plurality of directional buttons configured to indicate, as the data, a direction from which the first movable cart approaches the second movable cart; and/or wherein the data indicating a first pose of the first movable cart and a second pose of the second movable cart includes user entered pose information.

4. The surgical robotic system according to any preceding claim, wherein the instructions, when executed by the processor, further cause the surgical robotic system to:
automatically command each of the first movable cart and the second movable cart to move through a plurality of translation trajectories in an orthogonal direction; and
receive user input, as the data, correlating with tracking movement of each of the first movable cart and the second movable cart through the plurality of translation trajectories.

5. The surgical robotic system according to any preceding claim, wherein the first movable cart and the second movable cart each further include a sensor configured to measure travel and direction of the respective movable cart, and
wherein the instructions, when executed by the processor, further cause the surgical robotic system to:
generate a movement path, as the data, of each of the first movable cart and the second movable cart based on the sensed travel and direction of each of the first movable cart and the second movable cart.

6. The surgical robotic system according to any preceding claim, wherein the first movable cart further includes a telescoping arm including:
a plurality of joints including a respective plurality of encoders configured to provide signal indicating a distance; and
a sensor configured to sense contact of a predefined spatial location of the second movable cart by the telescoping arm, and
wherein the instructions, when executed by the processor, further cause the surgical robotic system to:
determine, as the data, the first pose of the first movable cart and the second pose of the second movable cart based on the sensed contact of the predefined spatial location of the second movable cart by the telescoping arm, the indicated distance, and the predefined spatial location.

7. The surgical robotic system according to claim 2, wherein the first movable cart includes a first end effector, and
wherein the second movable cart includes a second end effector, and
wherein the instructions, when executed by the processor, further cause the surgical robotic system to:
display, on the display, a predefined trajectory on the display for each of the first end effector and the second end effector; and
receive input, as the data, correlating with tracking the predefined trajectory for each of the first end effector and the second end effector.

8. The surgical robotic system according to any preceding claim, further comprising an imaging device configured to capture images of a surgical site, including a first end effector of the first movable cart and a second end effector of the second movable cart,
wherein the data includes the captured image; and
wherein the instructions, when executed by the processor, further cause the surgical robotic system to:
identify the first end effector and the second end effector based on a computer vision algorithm;
determine a relative pose between the first end effector and the second end effector based on the computer vision algorithm;
determine the pose of the first movable cart and the second movable cart based on inverse kinematic mapping of the determined relative pose between the first end effector and the second end effector; and
determine a position registration of the first cart based on the determined pose of the first movable cart and the second movable cart.

9. The surgical robotic system according to any preceding claim, wherein the first movable cart further includes a distance sensor configured to sense a distance between each of the movable carts, and
wherein the instructions, when executed by the processor, further cause the surgical robotic system to:
determine, as the data, the first pose of the first movable cart and the second pose of the second movable cart based on the sensed distance.

10. The surgical robotic system according to any preceding claim, further comprising an imaging device configured to capture an image including each of the first movable cart and the second movable cart, wherein each of the first movable cart and the second movable cart includes a marker, and
wherein the instructions, when executed by the processor, further cause the surgical robotic system to:
detect each of the markers in the image;
generate a point cloud of the first movable cart and the second movable cart in an operating room; and
identify each of the first movable cart and the second movable cart using image segmentation,
wherein the data includes the point cloud and the identified first movable cart and the second movable cart.

11. The surgical robotic system according to any preceding claim, wherein the first movable cart and the second movable cart each includes a spatial position sensor configured to sense a spatial position of the respective movable cart, and
wherein the instructions, when executed by the processor, further cause the surgical robotic system to:
sense, by the spatial position sensor, the spatial position of the first movable cart and the second movable cart; and
determine, as the data, the first pose of the first movable cart and the second pose of the second movable cart based on the sensed spatial position of the first movable cart and the second movable cart.

12. A computer-implemented method for controlling a surgical robotic system, the method comprising:
receiving data indicating a first pose of a first movable cart and a second pose of a second movable cart, wherein each of the movable carts includes a robotic arm; and
determining a relative location between the first movable cart and the second movable cart based on the first pose and the second pose; preferably further comprising:
determining a position registration of the first cart based on the relative location between the first movable cart and the second movable cart; and
displaying a graphical representation of the first movable cart based on the determined position registration on a display of a surgical console, wherein the display is configured to display a graphical user interface having a plurality of graphical representations, each of which corresponds to the first movable cart and the second movable cart.

13. The method according to claim 12, further comprising:
automatically commanding each of the first movable cart and the second movable cart to move through a plurality of translation trajectories in an orthogonal direction; and
receiving user input, as the data, correlating with tracking movement of each of the first movable cart and the second movable cart through the plurality of translation trajectories and/or further comprising:
contacting a predefined spatial location of the second movable cart by a telescoping arm of the first movable cart, the telescoping arm including:
a plurality of joints including a respective plurality of encoders configured to provide a signal indicating a distance; and
a sensor configured to sense contact of the telescoping arm with another movable cart,
sensing, by the sensor, the contact of the telescoping arm with the second movable cart;
sensing, by the plurality of encoders, the distance between the first movable cart and the second movable cart; and
determining, as the data, the first pose of the first movable cart and the second pose of the second movable cart based on the sensed distance and the predefined spatial location.

14. The method according to claim 12 or claim 13, further comprising:
capturing an image of a surgical site by an imaging device as the data, the image including a first end effector of the first movable cart and a second end effector of the second movable cart,
identifying the first end effector and the second end effector based on a computer vision algorithm;
determining a relative pose between the first end effector and the second end effector based on the computer vision algorithm;
determining the pose of the first movable cart and the second movable cart based on inverse kinematic mapping of the determined relative pose between the first end effector and the second end effector; and
determining a position registration of the first cart based on the determined pose of the first movable cart and the second movable cart; and/or further comprising:
sensing, by a spatial position sensor, the spatial position of each of the first movable cart and the second movable cart, wherein the spatial position sensor is; and
determine, as the data, the first pose of the first movable cart and the second pose of the second movable cart based on the sensed spatial position of the first movable cart and the second movable cart; preferably further comprising:
sensing by first sensor a first travel and first direction of the first movable cart;
sensing by second sensor a second travel and second direction of the second movable cart; and
generating a movement path, as the data, of each of the first movable cart and the second movable cart based on the sensed travel and direction of each of the first movable cart and the second movable cart.

15. A non-transitory computer-readable medium storing instructions which, when executed by a processor, cause the processor to perform a method for controlling a surgical robotic system, comprising:
receiving data indicating a first pose of a first movable cart and a second pose of a second movable cart, wherein each of the movable carts includes a robotic arm; and
determining a relative location between the first movable cart and the second movable cart based on the first pose and the second pose.
